# EUROPEAN PATENT APPLICATION

(11) **EP 4 406 537 A1**
(43) Date of publication of application: **31.07.2024**
(21) Application number: 22872030.6
(22) Date of filing: 21.09.2022
(51) Int. Cl.: A61K 31/201, A61K 31/194, C07C 55/21, C07C 55/28, C07C 57/13, A61P 3/06, A61P 3/00, A61P 9/10

(54) **LONG-CHAIN COMPOUND AND USE THEREOF**

(30) Priority: 23.09.2021 CN 202111116447
(71) Applicant: Burgeon Therapeutics Co., Ltd., Shanghai 201210 (CN)
(72) Inventor: NAN, Fajun, Shanghai 201210 (CN); LI, Jingya, Shanghai 201210 (CN); ZHANG, Mei, Shanghai 201210 (CN); XIE, Zhifu, Shanghai 201210 (CN); ZHANG, Yangming, Shanghai 201210 (CN)
(74) Representative: Lavoix
(86) International application number: PCT/CN2022/120315
(87) International publication number: WO 2023/045985

(57) **Abstract**

Disclosed are a long-chain compound and the use thereof. The structure of the long-chain compound is as shown in formula (I). The compound can directly inhibit ACLY, has a significant therapeutic effect on metabolic diseases represented by hypercholesterolemia and non-alcoholic steatohepatitis, and can be used for developing and preparing therapeutic drugs for metabolic diseases such as hypercholesterolemia and non-alcoholic steatohepatitis.

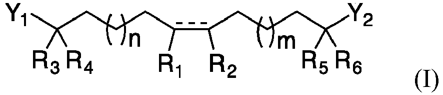

## Description

### Technical field

The present invention relates to a new class of long-chain fatty acid ACLY inhibitors, a pharmaceutically acceptable salt, pharmaceutically acceptable ester, crystalline hydrate, solvate thereof or mixtures thereof, a pharmaceutical composition containing such inhibitors, and use in the preparation of therapeutic drugs for metabolic diseases such as hypercholesterolemia, non-alcoholic steatohepatitis (NASH), liver fibrosis and the like.

### Background technique

Acetyl-CoA produced by the mitochondrial tricarboxylic acid cycle (TCA cycle) is a source substrate for the de novo synthesis of endogenous cholesterol and triglycerides. Acetyl-CoA generated by the tricarboxylic acid cycle usually cannot directly pass through the mitochondrial membrane to enter the cytoplasm. Instead, citric acid is generated in the mitochondria through the catalysis of citrate synthase; citric acid enters the cytoplasm through the mitochondrial membrane with the help of the tricarboxylic acid transporter, and then cleaved into acetyl-CoA and oxaloacetate by ATP-dependent citrate lyase (adenosine triphosphate citrate lyase, ACLY) distributed in the endoplasmic reticulum. Among them, oxaloacetate uses NADH to generate malic acid, which further generates pyruvate with the help of NAPDH. Malic acid and pyruvate can be transported back to the mitochondria through their respective transporters; acetyl-CoA can be used as a substrate to directly participate in the de novo synthesis of triglycerides and cholesterol. Various metabolic diseases such as non-alcoholic steatohepatitis (NASH), hypercholesterolemia, and atherosclerosis are related to abnormally elevated levels of hepatic lipid synthesis. Therefore, inhibiting the activity of ACLY can significantly reduce de novo lipid synthesis of hepatocyte lipids, and ACLY inhibitors can be used to prepare therapeutic drugs for metabolic diseases. It is known that Bempedoic acid (ETC-1002) is the first globally marketed small molecule inhibitor of ACLY and is mainly used for the treatment of statin-intolerant heterozygous familial hypercholesterolemia. Moreover, benzenesulfonamides and other ACLY inhibitors have also been verified to have lipid-lowering pharmacological activity at the cellular level and preclinical animal level.

Therefore, ACLY inhibitors need to be investigated for the treatment of metabolic diseases such as hypercholesterolaemia, non-alcoholic steatohepatitis (NASH) and liver fibrosis.

### Summary of the invention

The object of the present invention is to provide a use of long-chain compounds for the treatment of cholesterolemia, non-alcoholic steatohepatitis (NASH), liver fibrosis, atherosclerosis and other diseases.

The first aspect of the present invention provides a use of a compound represented by general formula (I), or a deuterated compound, stereoisomer, enantiomer, diastereomer, racemate or pharmaceutically acceptable salt thereof or mixtures of two or more compounds of the compounds represented by general formula (I) for the preparation of a medicament for the treatment of metabolic diseases; or for the preparation of a medicament for the treatment of atherosclerosis, 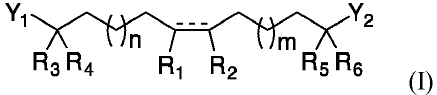
wherein - - - represents a double bond or a single bond;
m and n are each independently 1, 2, 3, 4, 5 or 6;
R₁, R₂, R₃, R₄, R₅ and R₆ are each independently H, C1-C4 alkyl, C2-C4 alkenyl, C2-C4 alkynyl, C3-C7 cycloalkyl, C3-C7 cycloalkenyl, phenyl or benzyl;
or R₁ and R₂, together with their adjacent carbon atoms, form a C3-C7 cycloalkyl or C3-C7 cycloalkenyl;
or R₅ and R₆, together with their adjacent carbon atom, form a C3-C7 cycloalkyl or C3-C7 cycloalkenyl;
or R₃ and R₄, together with their adjacent carbon atom, form a C3-C7 cycloalkyl or C3-C7 cycloalkenyl;
Y₁ and Y₂ are each independently selected from the group consisting of -OH, -COOH, - COCoA, and -COOR₇; wherein R₇ is methyl, ethyl or 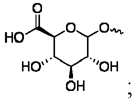 CoA is as follows:

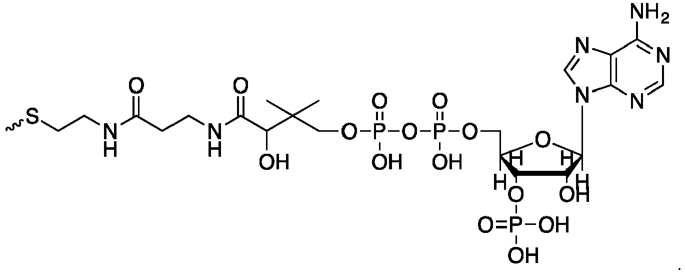

In another preferred example, - - - represents a double bond, and the configuration of the double bond is cis or trans.

In another preferred example, the compound has a structure represented by general formula II:

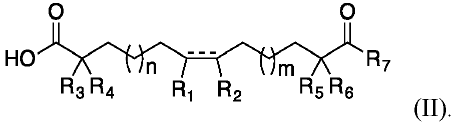

In another preferred example, the compound has a structure represented by general formula III:

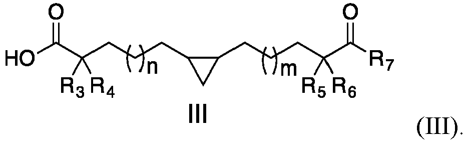

In another preferred example, wherein the compound has a structure represented by general formula IV: 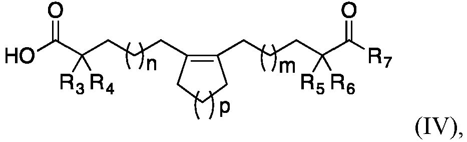
wherein p is 0, 1, 2 or 3.
In another preferred example, R₁, R₂, R₃, R₄, R₅, and R₆ are each independently H, C1-C4 alkyl, deuterated C1-C4 alkyl, C2-C4 alkenyl, C2-C4 alkynyl , C3-C7 cycloalkyl, deuterated C3-C7 cycloalkyl, C3-C7 cycloalkenyl, phenyl or benzyl;
or R₁ and R₂, together with their adjacent carbon atoms, form a deuterated C3-C7 cycloalkyl, C3-C7 cycloalkyl or C3-C7 cycloalkenyl;
or R₅ and R₆, together with their adjacent carbon atom, form a deuterated C3-C7 cycloalkyl, C3-C7 cycloalkyl or C3-C7 cycloalkenyl;
or R₃ and R₄, together with their adjacent carbon atom, form a deuterated C3-C7 cycloalkyl, C3-C7 cycloalkyl or C3-C7 cycloalkenyl.

In another preferred example, R₁ and R₂ are each independently H, methyl, ethyl, deuterated methyl, deuterated ethyl, deuterated propyl or propyl, preferably R₁ and R₂ are H or R₁ and R₂ are methyl; or R₁ and R₂, together with their adjacent carbon atoms, form a C3-C6 cycloalkyl, C3-C5 cycloalkyl or C3-C4 cycloalkyl.

In another preferred example, R₃ and R₄ are each independently H, methyl, ethyl, deuterated methyl, deuterated ethyl, deuterated propyl or propyl, preferably R₃ and R₄ are H or R₃ and R₄ are methyl; or R₃ and R₄, together with their adjacent carbon atom, form a C3-C6 cycloalkyl, C3-C5 cycloalkyl or C3-C4 cycloalkyl.

In another preferred example, R₅ and R₆ are each independently H, methyl, ethyl, deuterated methyl, deuterated ethyl, deuterated propyl or propyl, preferably R₅ and R₆ are H or R₅ and R₆ are methyl; or R₅ and R₆, together with their adjacent carbon atom, form a C3-C6 cycloalkyl, C3-C5 cycloalkyl or C3-C4 cycloalkyl.

In another preferred example, Y₁ and Y₂ are each independently -COOH, -COCoA or - COOR₇; wherein R₇ is methyl, ethyl or 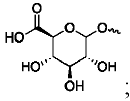 CoA is as follows:

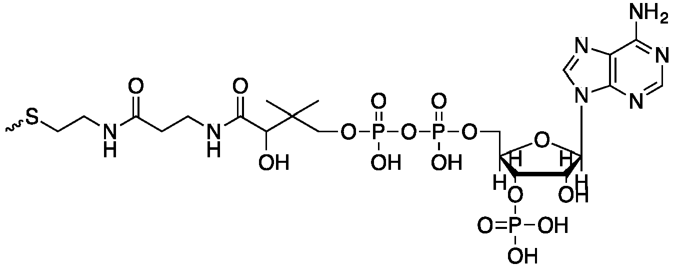

In another preferred example, the compound is selected from the following group:

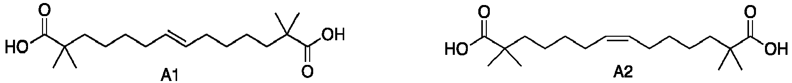

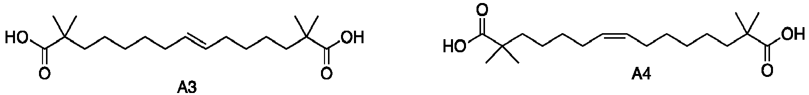

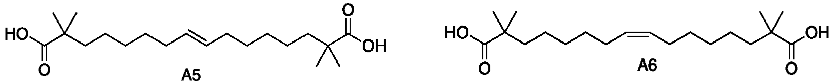

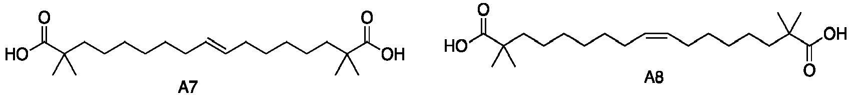

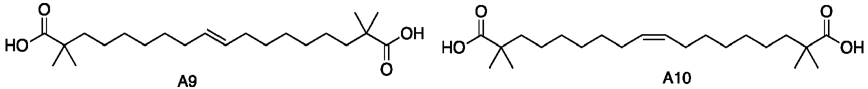

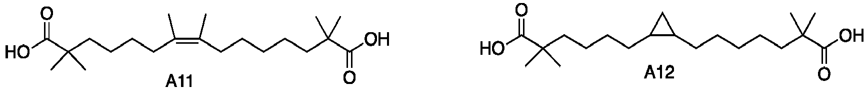

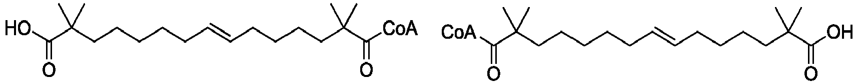

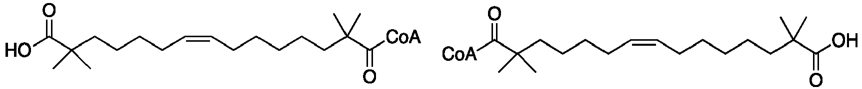

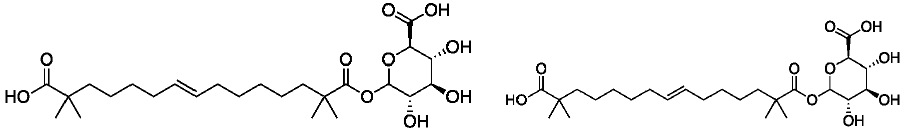

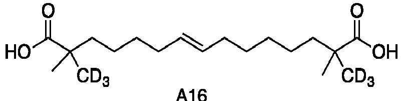

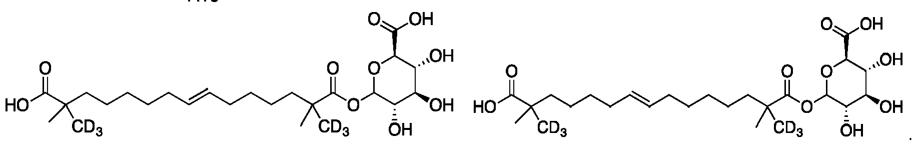

In another preferred example, the compound is also selected from the following group:

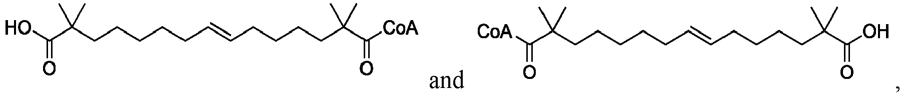

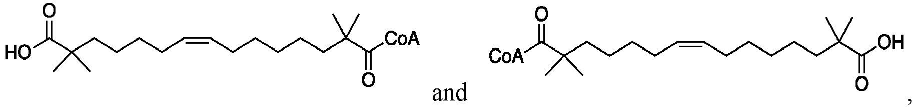

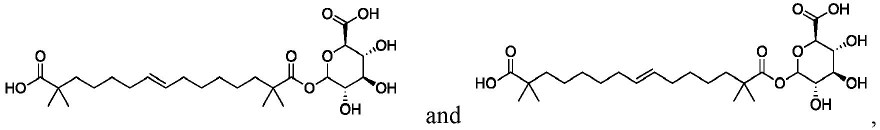

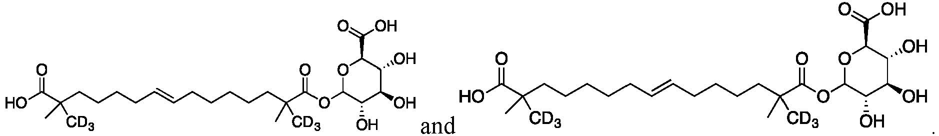

In another preferred example, the metabolic disease is selected from the group consisting of hypercholesterolemia, non-alcoholic steatohepatitis (NASH), and liver fibrosis.

In another preferred example, the atherosclerosis is caused by hypercholesterolemia.

The second aspect of the present invention provides a compound represented by general formula (I), or a deuterated compound, stereoisomer, enantiomer, diastereomer, racemate or pharmaceutically acceptable salt thereof or a mixture of two or more compounds of the compound represented by general formula (I), 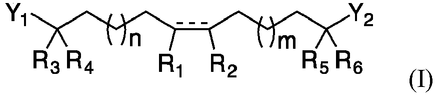
wherein - - - represents a double bond or a single bond;
m and n are each independently 1, 2, 3, 4, 5 or 6;
R₁, R₂, R₃, R₄, R₅ and R₆ are each independently H, C1-C4 alkyl, C2-C4 alkenyl, C2-C4 alkynyl, C3-C7 cycloalkyl, C3-C7 cycloalkenyl, phenyl or benzyl;
or R₁ and R₂, together with their adjacent carbon atoms, form a C3-C7 cycloalkyl or C3-C7 cycloalkenyl;
or R₅ and R₆, together with their adjacent carbon atom, form a C3-C7 cycloalkyl or C3-C7 cycloalkenyl;
or R₃ and R₄, together with their adjacent carbon atom, form a C3-C7 cycloalkyl or C3-C7 cycloalkenyl;
Y₁ and Y₂ are each independently selected from the group consisting of -OH, -COOH, - COCoA, and -COOR₇; wherein R₇ is methyl, ethyl or 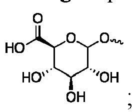 CoA is as follows:

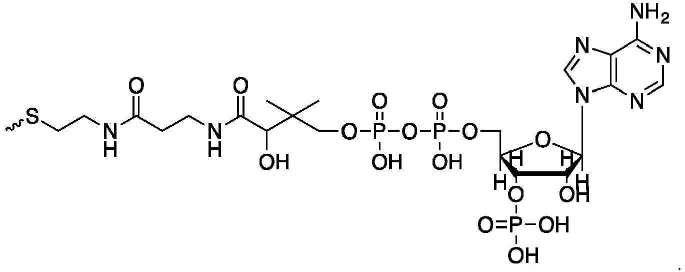

In another preferred example, the compound has a structure represented by general formula II, III or IV: 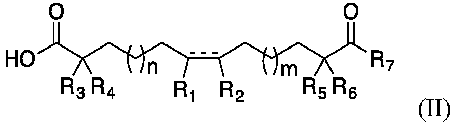 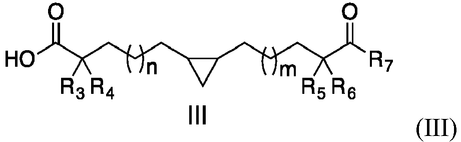 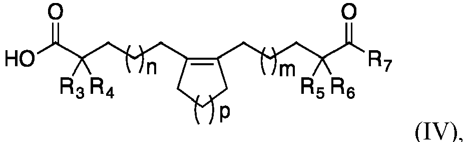 wherein p is 0, 1, 2 or 3.

In another preferred example, R₁, R₂, R₃, R₄, R₅ and R₆ are each independently H, C1-C4 alkyl, deuterated C1-C4 alkyl, C2-C4 alkenyl, C2-C4 alkynyl , C3-C7 cycloalkyl, C3-C7 cycloalkenyl, phenyl or benzyl;
or R₁ and R₂, together with their adjacent carbon atoms, form a deuterated C3-C7 cycloalkyl, C3-C7 cycloalkyl or C3-C7 cycloalkenyl;
or R₅ and R₆, together with their adjacent carbon atom, form a deuterated C3-C7 cycloalkyl,C3-C7 cycloalkyl or C3-C7 cycloalkenyl;
or R₃ and R₄, together with their adjacent carbon atom, form a deuterated C3-C7 cycloalkyl,C3-C7 cycloalkyl or C3-C7 cycloalkenyl.

In another preferred example, the compound is selected from the following group:

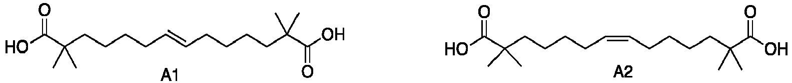

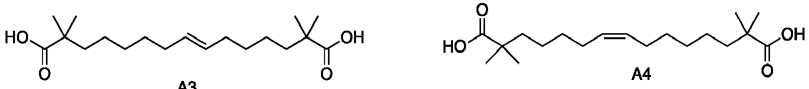

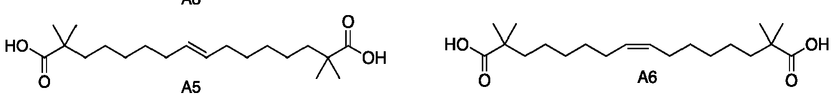

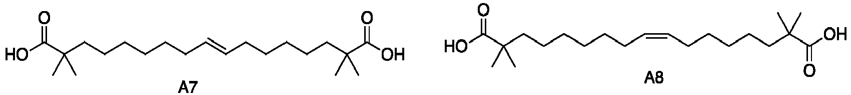

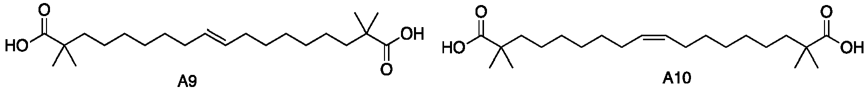

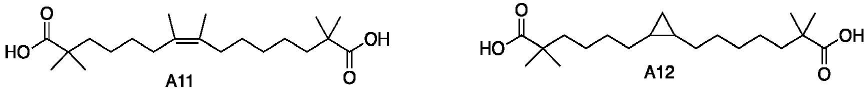

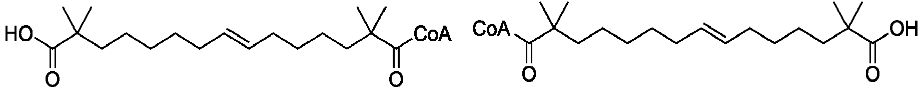

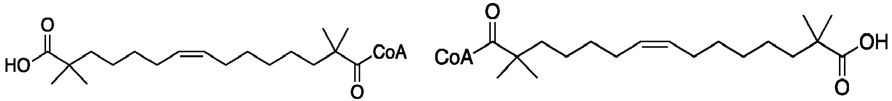

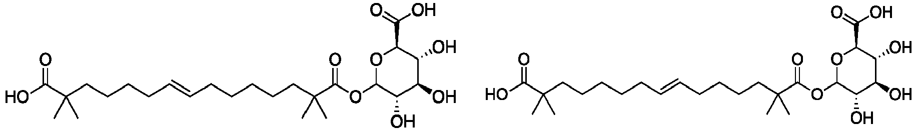

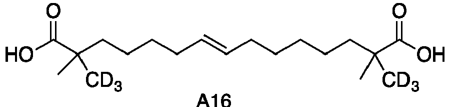

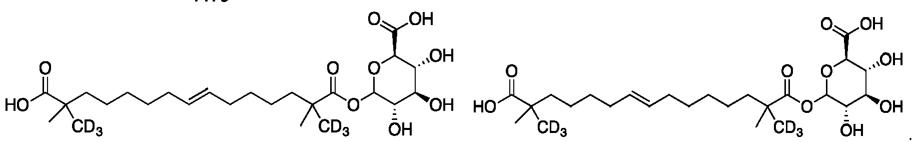

In another preferred example, the compound is also selected from the following group:

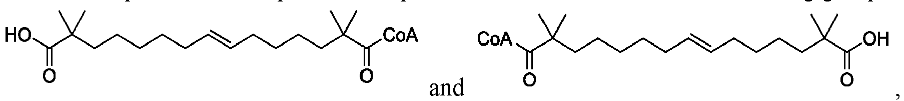

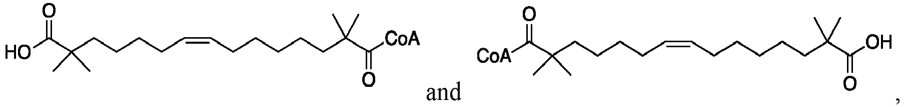

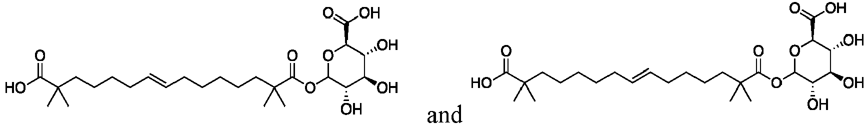

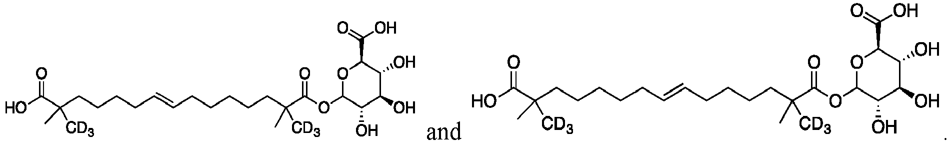

The third aspect of the present invention provides a pharmaceutical composition, comprising the compound according to the first aspect, or the deuterated compound, stereoisomer, enantiomer, diastereomer, racemate or pharmaceutically acceptable salt thereof or a mixture of two or more compounds in the compound represented by general formula (I); and
a pharmaceutically acceptable salt.

The dosage form of the pharmaceutical composition according to the present invention can be varied, including but not limited to: a tablet, capsule, granule, syrup, solution, suspension or aerosol.

The fourth aspect of the present invention provides a method for treating metabolic diseases, comprising administering to a subject in need the compound according to the second aspect, or the deuterated compound, stereoisomer, enantiomer, diastereomer, racemate or pharmaceutically acceptable salt thereof or a mixture of two or more compounds in the compound represented by general formula (I) or the pharmaceutical composition according to the third aspect.

In another preferred example, the metabolic disease is selected from the group consisting of hypercholesterolemia, non-alcoholic steatohepatitis (NASH), and liver fibrosis.

The fifth aspect of the present invention provides a method for treating atherosclerosis, comprising administering to a subject in need the compound according to the second aspect, or the deuterated compound, stereoisomer, enantiomer, diastereomer, racemate or pharmaceutically acceptable salt thereof or a mixture of two or more compounds in the compound represented by general formula (I) or the pharmaceutical composition according to the third aspect.

In another preferred example, the atherosclerosis is caused by hypercholesterolemia.

### Beneficial effects

Chinese Patent Application No. 201610105067 (CN107118098) discloses a type of fatty acid compound, preparation method and use thereof. This type of compound has the activity of activating APMK and has the ability to inhibit glucose output of mouse primary liver cells, and can be used to prepare a medicament for treating obesity or diabetes.

The inventor accidentally discovered that this type of compound has the activity of inhibiting ACLY. Further pharmacological activity evaluation *in vivo* and *in vitro* show that the representative compound A3 can inhibit the de novo synthesis of lipids in primary hepatocytes, improve hyperlipidemia and hypercholesterolemia in golden hamsters and ApoE^{-/-} mice, and significantly improve NASH symptoms induced by high-fat, high-sugar, high-cholesterol diet in *ob*/*ob* mice and crab-eating Macaque. The research results also suggest that the in vivo activity of compounds with a double bond in the trans configuration is better than that of compounds with a double bond in the cis configuration; moreover, the plasma exposure of Compound A3 is more than 4 times higher than that of Bempedoic acid at the same oral dose, suggesting that compound A3 has better therapeutic effect.

In summary, this series of compounds disclosed in the present invention have good ACLY inhibitory activity, can inhibit the de novo synthesis of triglycerides and cholesterol in primary hepatocytes, and significantly improve hyperlipidemia in golden hamsters and spontaneous elderly rhesus monkeys. Outstanding therapeutic effects have been shown in both NASH models of rodent and non-human primate.

As a new type of ACLY small molecule inhibitor, this class of compounds has significant therapeutic effects on metabolic diseases represented by hypercholesterolemia and non-alcoholic steatohepatitis, and can be used to develop therapeutic drugs for metabolic diseases such as hypercholesterolemia and non-alcoholic steatohepatitis.

It should be understood that within the scope of the present invention, the above-mentioned technical features of the present invention and the technical features specifically described below (such as examples) can be combined with each other to form new or preferred technical solutions. Each feature disclosed in the specification may be replaced by any alternative feature serving the same, equivalent or similar purpose. Due to space limitations, they will not be described one by one herein.

### Description of drawings

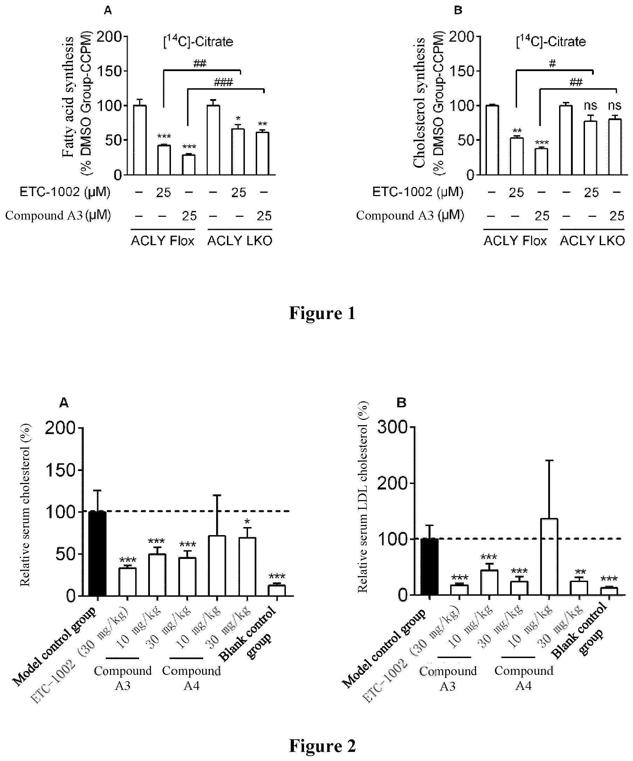

Figure 1 shows the evaluation of the inhibitory effect of compound A3 on de novo lipid synthesis in primary hepatocytes; wherein, A: investigation on the inhibitory effect of compound A3 on de novo triglyceride synthesis in the hepatocytes of WT mice and ACLY knockout mice; B: investigation on the inhibitory effect of compound A3 on de novo triglyceride synthesis in hepatocytes of WT mice and ACLY knockout mice. Compared with the control group, *P<0.05, **P<0.01, ***P<0.001. Compared with the corresponding dose group, ^{#}P<0.05, ^{##}P<0.01, ^{###}P<0.001.
Figure 2 shows the evaluation of the reducing effect of compound A3 and A4 on the serum total cholesterol and low-density lipoprotein cholesterol content of golden hamsters after multiple administrations, wherein, *, compared with the model control group, P < 0.05; **, compared with the model control group, P<0.01; ***, compared with the model control group, P<0.001.

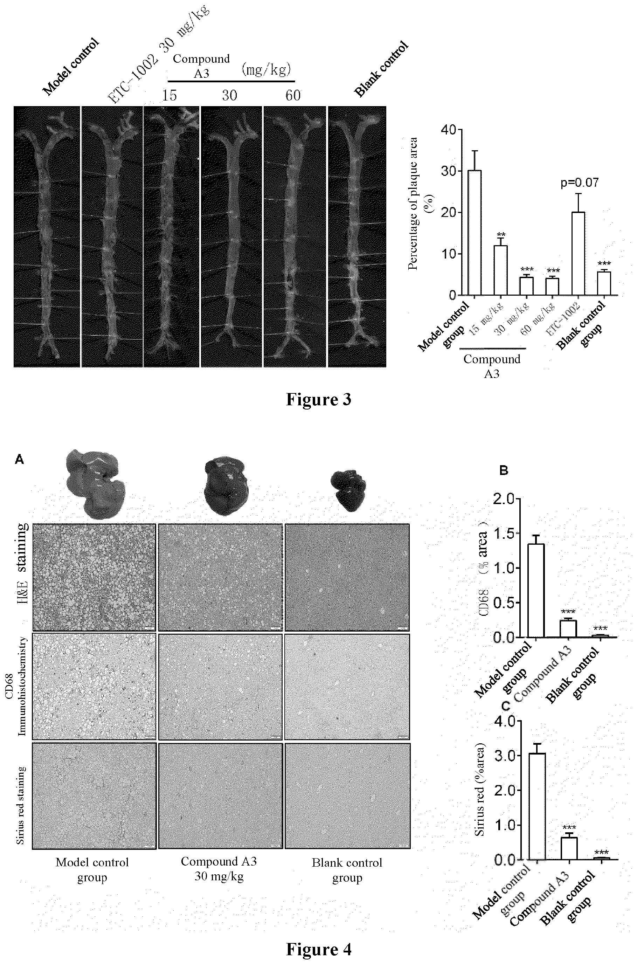

Figure 3 shows the effect of long-term administration of tested compound A3 on aortic plaque formation in ApoE^{-/-} mice, wherein, *, compared with the model control group, P<0.05; **, compared with the model control group, P<0.01; ***, compared with the model control group, P<0.001.
Figure 4 shows the evaluation of the therapeutic effect of compound A3 on NASH in *ob*/*ob* mice, wherein, A. liver appearance of *ob*/*ob* mice and liver pathological analysis results after long-term administration of the compound (n=8); B. Statistical graph of pathologic analysis of CD68 immunohistochemistry; C: Statistical graph of pathologic analysis of Sirius red staining; wherein, *, compared with the model control group, P<0.05; **, compared with the model control group, P<0.01; ***, compared with the model control group, P<0.001.

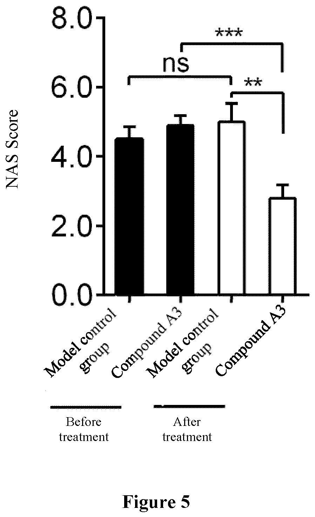

Figure 5 shows the changes in liver NAS score after long-term administration of compound A3, wherein, *, compared with the model control group, P < 0.05; **, compared with the model control group, P < 0.01; ***, compared with the model control group, P<0.001.

### Detailed description of the invention

Based on extensive and intensive studies, the inventor of the present application found that the compound of the present application has ACLY inhibitory activity. This type of compound can inhibit the de novo synthesis of lipids in primary hepatocytes and has shown therapeutic effects on various animal models of metabolic diseases. It can be further developed into a therapeutic drug for metabolic diseases such as hypercholesterolemia and non-alcoholic steatohepatitis, and can also be used to treat atherosclerosis. On this basis, the present invention was completed.

### Term

In the present invention, unless otherwise specified, the terms used have their ordinary meanings known to those skilled in the art.

In the present invention, the term "C₁-C₆" means there are 1, 2, 3 or 4 carbon atoms. The term "C₃-C₇" means there are 3, 4, 5, 6 or 7 carbon atoms, and so on.

In the present invention, the term "alkyl" refers to a saturated linear or branched hydrocarbon moiety. For example, the term "C1-C4 alkyl" refers to a linear or branched alkyl having 1 to 4 carbon atoms, including, but not limited to, methyl, ethyl, n-propyl, isopropyl, n-butyl, isobutyl, sec-butyl, and tert-butyl.

In the present invention, the term "alkenyl" refers to a linear or branched hydrocarbon moiety containing at least one double bond. For example, the term "C2-C4 alkenyl" refers to a linear or branched hydrocarbon group having 2 to 4 carbon atoms and containing one double bond, including, but not limited to, vinyl, propenyl, butenyl, and isobutenyl.

In the present invention, the term "alkynyl" refers to a linear or branched alkynyl containing one triple bond, including, but not limited to, ethynyl, propynyl, butynyl, isobutynyl, etc.

In the present invention, the term "cycloalkyl" refers to a saturated cyclic hydrocarbon moiety. For example, the term "C3-C7 cycloalkyl" refers to a cyclic alkyl group having 3 to 7 carbon atoms in the ring, including, but not limited to, cyclopropyl, cyclobutyl, cyclopentyl, cyclohexyl, etc.

In the present invention, the term "cycloalkenyl" refers to a cyclic hydrocarbon moiety containing at least one double bond. For example, the term "C3-C7 cycloalkenyl" refers to a cyclic alkenyl having 3 to 7 carbon atoms in the ring, including, but not limited to, cyclopropenyl, cyclobutenyl, cyclopentenyl, cyclohexenyl, and the like.

The pharmaceutically acceptable salt of the present invention may be a salt formed by an anion and a positively charged group on the compound of formula I. Suitable anions are chloride ion, bromine ion, iodine ion, sulfate, nitrate, phosphate, citrate, methanesulfonate, trifluoroacetate, acetate, malate, tosylate, tartrate, fumarate, glutamate, glucuronate, lactate, glutarate or maleate. Similarly, the salt can be formed from a cation with negatively charged group on compounds of formula I. Suitable cations include sodium, potassium, magnesium, calcium and ammonium ions, such as tetramethylammonium ion.

The present invention will be further described below in conjunction with specific examples. It should be understood that these examples are only used to illustrate the invention and are not intended to limit the scope of the invention. Experimental methods without specifying specific conditions in the following examples are usually carried out according to conventional conditions (such as the conditions described in Sambrook et al., Molecular Cloning: Laboratory Manual (New York: Cold Spring Harbor Laboratory Press, 1989)) or according to manufacturing conditions recommended by the manufacturer. Unless otherwise stated, percentages and parts are by weight.

Unless otherwise defined, all technical and scientific terms used herein have the same meaning as familiar to one skilled in the art. In addition, any methods and materials similar or equivalent to those described herein can be used in the method of the present invention. The preferred implementation methods and materials described herein are for demonstration purposes only.

### Example 1: Compound synthesis and characterization

### Synthesis of Compound A4

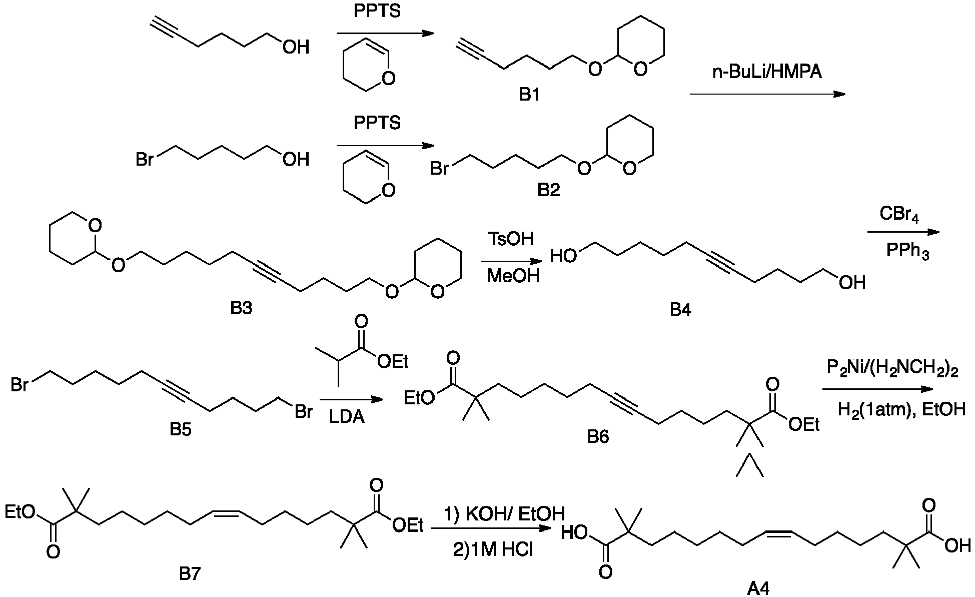

1. Compound 5-yne-1-hexanol (9g, 91mmol) and pyridinium p-toluenesulfonate (25.1g, 100mmol) were dissolved in 100mL DCM, tetrahydropyran (12.5mL, 126mmol) was added, and the mixture was stirred at room temperature overnight. TLC monitored the reaction to be complete. Most of the DCM is removed and about 30 mL is retained. About 100 mL of diethyl ether was added and stirred for 0.5 h. The solid was filtered out and washed with diethyl ether once. The filtrate was concentrated and passed through the column (PE/EA (volume ratio) = 50/1) to obtain product B1, oily substance, 18g, yield >100%.
2. Compound 5-bromo-1-pentanol (15.1g, 91mmol) and pyridinium p-toluenesulfonate (25.1g, 100mmol) were dissolved in 100mL DCM, tetrahydropyran (12.5mL, 126mmol) was added, and the mixture was stirred at room temperature overnight. TLC monitored the reaction to be complete. Most of the DCM is removed and about 30 mL is retained. About 100 mL of diethyl ether was added and stirred for 0.5 h. The solid was filtered out and washed with diethyl ether once. The filtrate was concentrated and passed through the column (PE/EA (volume ratio) = 50/1) to obtain product B2, 18g, yield >79%.
3. Compound B2 (5.4g, 30mmol) was dissolved in 40mL anhydrous THF, cooled to -40 °C, n-butyllithium (19mL, 30.4mmol) was added dropwise (about 0.5h), then 10mL HMPA was added, and stirred at this temperature. After 1 h, a solution of compound B2 (7.78 g, 31 mmol) in 10 mL THF was added. After 1 hour at this temperature, the mixture was slowly warmed to room temperature and stirred overnight, and then quenched by adding a saturated ammonium chloride solution and separated. The aqueous phase was extracted once with ethyl acetate. The combined organic phases was washed with water and saturated brine, dried, concentrated and passed through the column (PE/EA (volume ratio) = 20/1→10/1→5/1) to obtain product B3, 8.5g, yield 80%.
4. Compound B3 (3g, 8.5mmol) was dissolved in 100mL methanol and 10mL, p-toluenesulfonic acid (300mg) was added, and the mixture was stirred at room temperature overnight. TLC monitored the reaction to be complete. The mixture was concentrated to remove most of the methanol, diluted with water, and extracted twice with diethyl ether. The combined organic phases was washed with water and saturated brine, dried, concentrated and passed through the column (PE/EA (volume ratio) = 2/1→1/1→1/ 2) to obtain product B4, 1.1g, yield 70%.
5. Compound B4 (1.1g, 5.9mmol) and carbon tetrabromide (5.95g, 17.9mmol) were dissolved in 30mL anhydrous DCM, cooled to 0 °C, and a 10mL triphenylphosphine (4.7g, 17.9mmol) solution in DCM was added dropwise and stirred at room temperature for 1 hour, the reaction was complete. The mixture was concentrated to about 15 mL, and 50 mL of diethyl ether was added and stirred for 0.5 h, filtered to remove the solid. The filtrate was concentrated and passed through the column with PE/EA = 30/1 to obtain bromide B5, 1.84 g, with a yield of 100%.
6. Compound B5 (1.84g, 5.9mmol) and ethyl isobutyrate (2.73g, 23.6mmol) were dissolved in 50mL of anhydrous THF, and cooled to 0 °C. LDA (15.7mL, 23.6mmol) was added dropwise (about 0.5h). The temperature was kept at this temperature for 1h, and then raised to room temperature. The mixture was stirred overnight. The reaction was monitored by TLC. The reaction mixture was quenched by adding saturated ammonium chloride solution and layered. The aqueous phase was extracted with EA twice. The combined organic phases was washed with water and saturated saline, dried, concentrated and passed through the column (PE/EA (v/v) = 50/1→20/1) to obtain the product B6, 1.8g, yield 80%.
7. Nickel acetate (749 mg, 3 mmol) was suspended in 20 mL of anhydrous ethanol under one atmosphere of H₂, sodium borohydride (114 mg, 3 mmol) was added rapidly, H₂ was displaced twice and the solution was darkened. After stirring for 15 min at room temperature, ethylenediamine (0.45 mL, 6 mmol) and then 10 mL of anhydrous ethanol solution of compound B6 (1.8 g, 4.7 mmol) were added. After stirring at room temperature for 2h, the reaction was completed by being monitored by TLC. The mixture was diluted by adding 50mL of ether, filtered through a pad of diatomaceous earth, and the filtrate was concentrated and passed through column (PE/EA (v/v) = 50/1) to give the product B7, 1.7g, in 95% yield.
8. Compound B7 (1.7 g, 4.45 mmol) was dissolved in 50 ml of ethanol, 10 mL of aqueous KOH (2 g, 35 mmol) was added, and the reaction was heated to reflux for 6 h. The reaction was monitored by TLC. After the mixture was cooled, most of the ethanol was removed through rotary evaporation. The residue was diluted with water (40 ml), and extracted with diethyl ether twice to remove impurities. The aqueous layer was adjusted to acidity with 2N HCl, extracted with DCM for 4 times, the organic phases were combined, washed with water and saturated saline, dried, concentrated and passed through the column (DCM/MeOH (v/v) = 100/1) to obtain A4, 1.1g.

**A4** ¹H NMR (600 MHz, DMSO) δ5.31-5.32(m, 2H), 1.96-1.98 (m, 4H), 1.40-1.44(m, 4H), 1.22-1.30 (m, 4H), 1.18-1.22 (m, 6H), 1.06 (s, 12H).

The following compounds were obtained by substituting different substrates and adopting a synthetic route similar to that of A4.

**A2** ¹H NMR (300 MHz, CD3Cl) δ5.31-5.32(m, 2H), 1.96-1.98 (m, 4H), 1.40-1.44(m, 4H), 1.18-1.22 (m, 8H), 1.06 (s, 12H)

**A6** ¹H NMR (300 MHz, CD3Cl) δ5.30-5.32(m, 2H), 1.96-1.98 (m, 4H), 1.40-1.45(m, 4H), 1.18-1.22 (m, 12H), 1.06 (s, 12H)

**A8** ¹H NMR (400 MHz, CDCl3) δ 5.40 - 5.29 (m, 2H), 2.05 - 1.93 (m, 4H), 1.57 - 1.48 (m, 4H), 1.39 - 1.22 (m, 14H), 1.19 (s, 6H), 1.18 (s, 6H).

A10 ¹H NMR (300 MHz, CD3Cl) δ5.30-5.32(m, 2H), 1.98-2.02(m, 4H), 1.48-1.53(m, 4H), 1.18-1.22 (m, 16H), 1.06 (s, 12H)

### Synthesis of compound A3

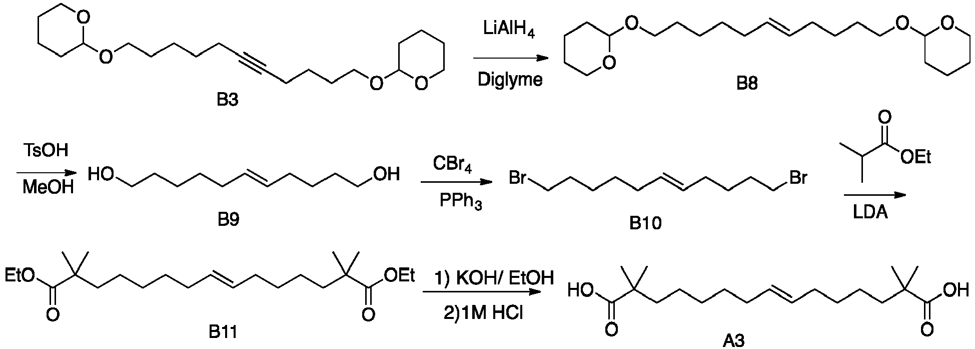

1. Under nitrogen protection, 350 mg of lithium aluminum tetrahydride (9.1 mmol) was dissolved in 10 ml of diethylene glycol diethyl ether, and 410 mg of B2 (1.1 mmol) was dissolved in 2 ml of diethylene glycol diethyl ether and reacted at 140 °C for 18 hours. TLC Monitor that the reaction is complete, then 30 ml of diethyl ether was added to dilute. Sodium sulfate decahydrate in batches was added under ice-water bath cooling conditions until there is no bubbling. The mixture was filtered through diatomaceous earth to remove the solid, washed with anhydrous diethyl ether, dry with anhydrous sodium sulfate, concentrated, and passed through the column (PE : EA=20: 1) to obtain 250 mg of product B8, a colorless liquid, with a yield of 62%.
2. Compound B8 (250 mg, 0.71 mmol) was dissolved in 10 mL of methanol and 11 mL, p-toluenesulfonic acid (25 mg) was added, and the mixture was stirred at room temperature overnight. TLC monitored the reaction to be complete. The reaction mixture was concentrated to remove most of the methanol, diluted with water, and extracted twice with diethyl ether. The combined the organic phases was washed with water and saturated brine, dried, concentrated and passed through the column (PE/EA (volume ratio) = 4/1) to obtain product B9, 100g. The yield is 75%.
3. Compound B9 (100 mg, 0.53 mmol) and carbon tetrabromide (360 mg, 1.1 mmol) were dissolved in 10 mL anhydrous DCM, cooled to 0°C, a 2 mL triphenylphosphine (256 g, 0.98 mmol) solution in DCM was added dropwise, and stirred at room temperature. After 1 hour, the reaction was complete. The mixture was concentrated and passed through the column (PE/EA (volume ratio) = 20/1) to obtain bromide B10, 142 mg, yield 87%.
4. Compound B10 (142 mg, 0.46 mmol) and ethyl isobutyrate (320 mg, 2.76 mmol) were dissolved in 10 mL anhydrous THF, cooled to 0°C, and LDA (1.84 mL, 2.76 mmol) was added dropwise (about 0.5 h) ). The mixture was at this temperature for 1 hour, then warmed to room temperature and stirred overnight. TLC monitored the reaction to be complete. A saturated ammonium chloride solution was added to quench the reaction and the mixture was separated. The aqueous phase was extracted twice with EA, and the combined organic phases was washed with water and saturated brine, dried, concentrated and passed through the column (PE/EA (volume ratio) = 50/1) to obtain product B11, 140 mg, yield 80%.
5. Compound B11 (107 mg, 0.28 mmol) was dissolved in 10 ml of ethanol, a 2 mL aqueous KOH solution (200 mg, 3.5 mmol) was added, and the mixture was heated to reflux for 6 h. TLC monitored the reaction to be complete. The mixture was cooled, subjected to rotary evaporation to remove most of the ethanol, diluted with water (10 ml) and extracted with diethyl ether twice to remove impurities. The aqueous layer was adjusted to acidity by adding 2N HCl and extracted with DCM for 4 times. The combined organic phases was washed with water and saturated brine, dried, concentrated and passed through the column (DCM/MeOH (volume ratio) = 100/1) to obtain 70 mg of compound A3.

A3 ¹H NMR (600 MHz, DMSO) δ5.35-5.36(m, 2H), 1.92-1.94 (m, 4H), 1.40-1.43 (m, 4H), 1.27-1.29 (m, 4H), 1.17-1.19 (m, 6H),1.18 ppm (s, 12H).

The following compounds were obtained by substituting different substrates and adopting a synthetic route similar to that of A3.

A1 ¹H NMR (400 MHz, CDCl3) δ 5.37 - 5.23 (m, 2H), 2.07 - 1.90 (m, 4H), 1.60 - 1.44 (m, 4H), 1.34 - 1.21 (m, 8H), 1.16 (s, 12H).

A5 ¹H NMR (400 MHz, CDCl3) δ 5.41 - 5.30 (m, 2H), 2.09 - 1.87 (m, 4H), 1.60 - 1.46 (m, 4H), 1.41 - 1.32 (m, 4H), 1.31 - 1.23 (m, 8H), 1.22 - 1.07 (s, 12H).

A7 ¹H NMR (400 MHz, CDCl3) δ 5.40 - 5.28 (m, 2H), 2.06 - 1.88 (m, 4H), 1.57 - 1.48 (m, 4H), 1.40 - 1.20 (m, 14H), 1.18 (s, 12H).

A9 ¹H NMR (400 MHz, CDCl3) δ 5.33 - 5.24 (m, 2H), 2.00 - 1.92 (m, 4H), 1.54 - 1.47 (m, 4H), 1.31 - 1.17 (m, 28H). 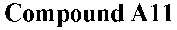 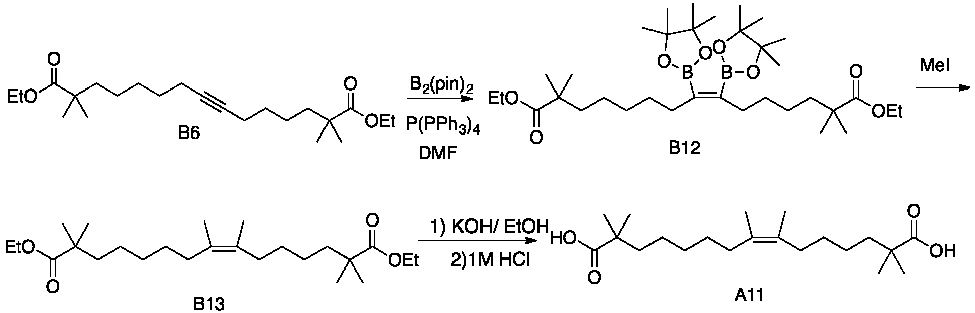
1. Pinacol diborate (483 mg, 1.9 mmol) and tetra-triphenylphosphoplatinum (70 mg) were placed in a 25 mL round-bottomed flask. After argon replacement three times, a solution of B6 (660 mg, 1.74 mmol) in 10 mL DMF was added dropwise and reacted at 80°C overnight. The mixture was diluted with water and extracted with diethyl ether. The combined organic phases was washed with water and saturated brine, dried, concentrated and passed through the column (PE/EA(volume ratio)=10/1) to obtain product B12, 1g, yield 90%.
2. Compound B12 (150 mg, 0.23 mmol), methyl iodide (0.1 mL) and triphenylphosphine (10 mg) were dissolved in 1 mL of dioxane. After argon gas replacement three times, 0.2 mL aqueous solution of palladium acetate (20 mg) and KOH (40 mg) were added and reacted at 90 °C for 12 hours. The mixture was diluted by adding water and ethyl acetate and extracted 3 times with ethyl acetate. The combined organic phases was washed with water and saturated brine, dried, concentrated and passed through the column (PE/EA=50/1) to obtain product B13, 30mg, yield 31%.
3. Compound B13 (30 mg, 0.07 mmol) was dissolved in 5 ml of ethanol, 1 mL aqueous solution of KOH (50 mg, 0.89 mmol) was added, and the mixture was heated and refluxed for 6 hours. TLC monitored the reaction to be complete. The mixture was cooled, subjected to rotary evaporation to remove most of the ethanol, diluted with water (4ml) and extracted twice with diethyl ether to remove impurities. The aqueous layer was adjusted to acidity by adding 2N HCl and extracted with DCM for 4 times. The combined organic phases was washed with water and saturated brine, dried, concentrated and passed through the column (DCM/MeOH(volume ratio)=100/1) to obtain A11, 15mg.

**A11** ¹H NMR (300 MHz, CDCl₃) δ 1.94-1.96 (m, 4H), 1.58(s, 6H), 1.48-1.50 (m, 4H), 1.25-1.33(m, 12H), 1.13 ppm (s, 12H)

### Synthesis of compound A12

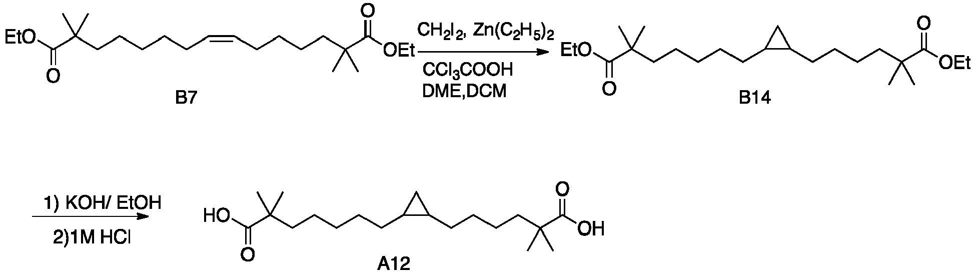

1. Under nitrogen protection, diethylzinc reagent (1 mL, 1 mmol) was dissolved in 1 mL of DCM, cooled to -40 °C for 5 min, then 0.5 mL of diiodomethane (0.54 g, 2 mmol) solution in DCM was added dropwise and reacted at -40 °C for 1h. Trichloroacetic acid (16 mg, 0.1 mmol) and DME (45 mg, 0.5 mmol) were added. After addition, the reaction was raised to -15°C for 1h. 0.5 mL B7 (193 mg, 0.5 mmol) solution in DCM was added and then stirred overnight at room temperature. Saturated ammonium chloride solution was added to quench the reaction and the mixture was extracted three times with ethyl acetate. The combined organic phases was washed with water and saturated brine, dried, concentrated and passed through the column (PE/EA(volume ratio)=50/1) to obtain product B14, 120mg, yield 60%.
2. Compound B14 (100mg, 0.25mmol) was dissolved in 5 ml of ethanol, 1 mL aqueous solution of KOH (10 mg, 0.18 mmol) was added, and the mixture was heated and refluxed for 6 hours. The mixture was cooled, subjected to rotary evaporation to remove most of the ethanol, diluted with water (4ml) and extracted twice with diethyl ether to remove impurities. The aqueous layer was adjusted to acidity by adding 2N HCl and extracted with DCM for 4 times. The combined organic phases was washed with water and saturated brine, dried, concentrated and passed through the column (DCM/MeOH(volume ratio)=100/1) to obtain A12, 65mg.

**A12** ¹H NMR (300 MHz, CDCl₃) δ 1.1.45-1.52 (m, 4H), 1.21-1.43(m, 16H), 1.18 (s, 12H), 0.65-0.70ppm (m, 2H)

### Synthesis of compound A13

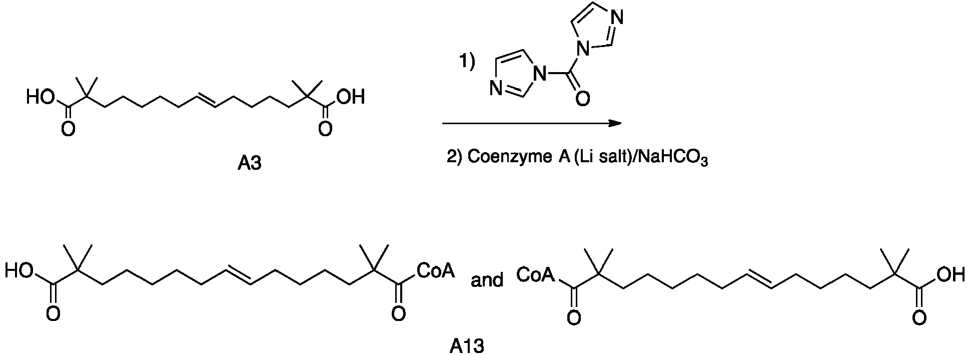

1. Compound A3 (30 mg) was dissolved in CH₂Cl₂ (5.0 mL), 1,1'-carbonyldiimidazole (50 mg, 30 mmol) was added and stirred at room temperature for 2 h. The reaction mixture was diluted by adding 5.0 mL of CH₂Cl₂, washed with water (10 mL each time, twice) and dried over anhydrous magnesium sulfate. The product was directly to the next step.
2. Under argon protection, the above imidazole intermediate was dissolved in dry THF (8.5 mL), and coenzyme A trilithium salt (20 mg, 0.026 mmol) was added dropwise to a solution of NaHCO₃ (0.1 M, 3 mL). The reaction solution was stirred at room temperature for 24 h. The reaction solution was extracted with ethyl acetate (2 × 3 mL). The aqueous layer was adjusted to pH 2 with 1 M HCl. The aqueous layer (~1 mL) was passed directly onto a column (SPE, Agilent HF Bond Elur C18 column, size: 5 gm/20 mL, USA), and the concentrations of the eluents were 0%, 5%, 10%, 20%, 30%, 40%, 50%, and 100% MeCN (10 mL each time). A13 was obtained and the structure was confirmed by1 H NMR spectroscopy and LR-ESI-MS.

Compound A13: Solvent systems on Zorbax-SB C18column, 4.6*50mm, 3.5 µ: Gradient, A:10mM NH₄COOH in H₂O, B: CH₃OH, 0 min: 50% A and 50% B; 0-30 min: A 50%→0%, B 50%→100%, 0.5mL/min. HPLC analysis: tr = 14.777 min and 16.074 min; white solid (13 mg, 42%); ¹H NMR (400 MHz, D₂O) δ 8.65 (s, 1H), 8.58(s, 1H), 8.30(s, 1H), 6.01(d, J = 5.4 Hz, 1H), 5.45-5.42(m, 2H), 4.75-4.74 (m, 2H), 4.48 (bs, 1H), 4.28(bs, 2H), 3.98 (s, 1H), 3.82-3.80 (m, 1H), 3.56-3.52(m, 1H), 3.40-3.38 (m, 2H), 3.20-3.18(m, 2H), 2.25-2.22(m, 2H), 2.33-2.30 (m, 2H), 1.78-1.74(m, 4H), 1.42-1.35(m,4H), 1.29-1.20 (m, 10H), 1.06 (s, 12H), 0.80 (s, 3H), 0.65 (s, 3H)ppm; LR-ESI-MS m/z calcd for C₄₀H₆₈N₇O₁₉P₃S [M-H]⁻ : 1074.3, found 1074.3

### Synthesis of compound A14

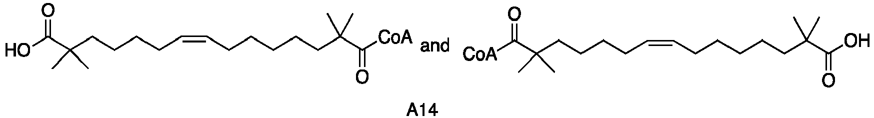

Using A4 as a substrate, A14 was obtained by referring to the synthesis method of compound A13. ¹H NMR (400 MHz, D₂O) δ 8.65 (s, 1H), 8.57(s, 1H), 8.30(s, 1H), 6.02(d, J = 5.4 Hz, 1H), 5.47-5.42(m, 2H), 4.75-4.73 (m, 2H), 4.49 (bs, 1H), 4.27(bs, 2H), 3.98 (s, 1H), 3.82-3.81 (m, 1H), 3.56-3.52(m, 1H), 3.40-3.38 (m, 2H), 3.20-3.18(m, 2H), 2.25-2.20(m, 2H), 2.33-2.29 (m, 2H), 1.78-1.75(m,4H), 1.42-1.35(m,4H), 1.29-1.19 (m, 10H), 1.06 (s, 12H), 0.80 (s, 3H), 0.65 (s, 3H)ppm; LR-ESI-MS m/z calcd for C₄₀H₆₈N₇O₁₉P₃S [M-H]⁻ : 1074.3, found 1074.3

### Synthesis of compound A15

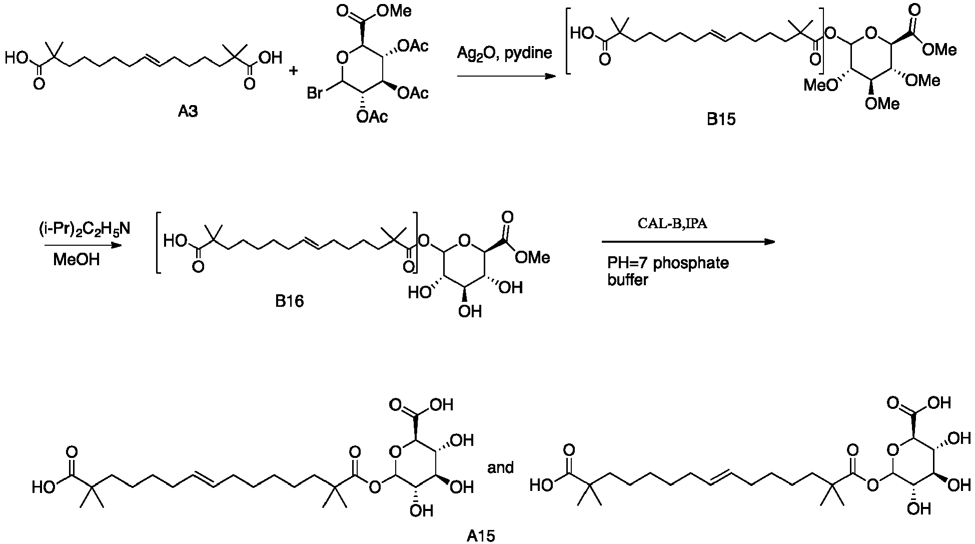

1. Compound A3 (326 mg, 1 mmol) and (2S,3R,4S,5S,6S)-2-bromo-6-(methoxycarbonyl)tetrahydro-2H-pyran-3,4,5-triyltriacetate (476 mg, 1.20 mmol) were dissolved in 10 ml of toluene, cooled to 0 °C, and silver oxide (925 mg, 4 mmol) and pyridine (0.35 ml, 4 mmol) were added and stirred overnight at room temperature. The mixture was filtered by diatomaceous earth pad, washed with toluene, concentrated and passed through a column (dichloromethane/methyl tert-butyl ether (volume ratio)=10/1) to obtain B15, 350mg.
2. B15 (350 mg, 0.54 mmol) was dissolved in 5 ml of methanol, cooled to 0 °C, and diisopropylethylamine (0.3 ml) was added and stirred at room temperature for 2 days. Then the mixture was concentrated and passed through the column (petroleum ether/ethyl acetate/isopropanol (volume ratio) = 50/50/6) to give 100 mg of compound B16.
3. B16 (60 mg, 0.17 mmol) was dissolved in isopropanol (0.5 ml), a phosphate buffer solution (5 ml, PH = 7) was added and then CAL-B (100 mg) was added. The mixture was stirred at room temperature for 3 hours. A small amount of isopropanol was removed and the mixture was filtered, washed with water and extracted with ethyl acetate twice. The combined organic phases was washed with water and saturated brine, dried, concentrated and passed through the reversed-phase column (MeOH/H₂O(volume ratio)=90/10) to obtain A15, 15mg.

Compound A15 ¹H NMR (400 MHz, CD3OD) δ 5.46 (d, J = 7.9 Hz, 1H), 5.40 - 5.35 (m, 2H), 3.80 (d, J = 9.5 Hz, 1H), 3.57 - 3.34 (m, 3H), 2.01 - 1.94 (m, 4H), 1.60 - 1.47 (m, 4H), 1.39 - 1.23 (m, 10H), 1.22 - 1.17 (m, 6H), 1.17 - 1.11 (m, 6H) ppm. LR-ESI-MS m/z calcd for C₂₅H₄₂O₁₀ [M-H]⁻: 501.2, found 501.2.

### Synthesis of compound A16

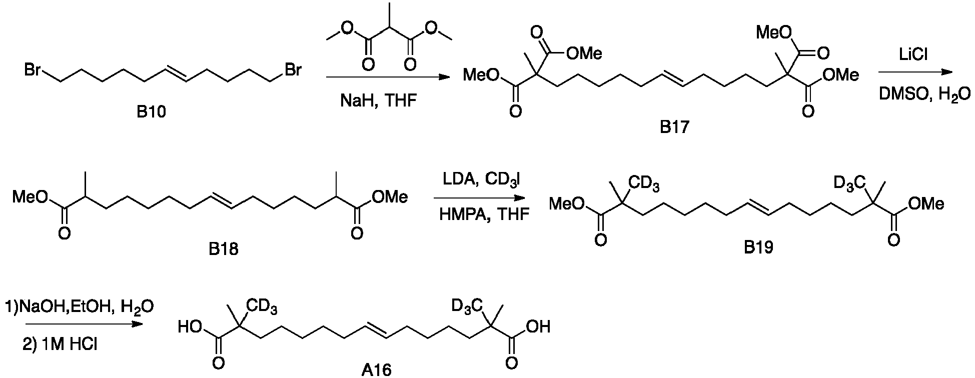

1. NaH (672 mg, 16.8 mmol) was suspended in 10 mL of THF, cooled to 0 °C, and dimethyl methylmalonate (2.35 g, 16 mmol) was added dropwise (about 0.5 h) and warmed to room temperature and stirred for 0.5 h. Compound B10 (730 mg, 2.3 mmol)/10mLTHF solution was then added slowly dropwise. Then the mixture was stirred at 60°C for 7h, the reaction was monitored by TLC. A saturated ammonium chloride solution was added to quench the reaction and the mixture was layered. The aqueous phase was extracted with EA twice. The combined organic phases was washed with water and saturated brine, dried, concentrated and passed through the column (PE/EA(volume ratio)=50/1) to obtain product B17, 510mg, yield 50%.
2. Compound B17 (300 mg, 0.68 mmol) was dissolved in 5 mL of DMSO, LiCl ((286 mg, 6.8 mmol) and H₂O (0.12 mL) were added and reacted by microwave heating to 180 °C for 5 min. The reaction is monitored by TLC until complete. The mixture was cooled and diluted with water. The aqueous phase was extracted 4 times with EA. The combined organic phases was washed with water and saturated brine, dried, concentrated and passed through the column (PE/EA(volume ratio)=50/1) to obtain product B18, 166mg, yield 75%.
3. Compound B17 (140 mg, 0.43 mmol) was dissolved in 5 mL of anhydrous THF, cooled to -78 °C, LDA (1.84 mL, 2.76 mmol) and HMPA (0.4 mL) were added dropwise and kept at this temperature for 1 h. Then CD₃I (0.16 mL, 2.58 mmol) was added dropwise, and the mixture was warmed to room temperature and stirred overnight. The reaction is monitored by TLC until complete. A saturated ammonium chloride solution was added to quench the reaction and the mixture was layered. The aqueous phase was extracted with EA twice. The combined organic phases was washed with water and saturated brine, dried, concentrated and passed through the column (PE/EA(volume ratio)=50/1) to obtain product B19, 77mg, yield 49%.
4. Compound B19 (77 mg, 0.21 mmol) was dissolved in ethanol (2 mL), 2 mL of aqueous NaOH (120 mg, 3 mmol) was added, and heated to reflux for 6h. The reaction is monitored by TLC until complete. The reaction mixture was cooled and subjected to rotary evaporation to remove most of the ethanol, diluted with water (10 ml) and extracted with diethyl ether twice to remove impurities. The aqueous layer was adjusted to acidity by adding 2N HCl and extracted with DCM for 4 times. The combined organic phases was washed with water and saturated brine, dried, concentrated and passed through the column (DCM/MeOH(volume ratio)=50/1) to obtain compound A16 50mg.
A16 ¹H NMR (400 MHz, CD₃Cl) 65.33-5.34(m, 2H), 1.96-1.99 (m, 4H), 1.51-1.55 (m, 4H), 1.35-1.42 (m, 4H), 1.22-1.28 (m, 6H),1.18 (s, 3H), 1.17 ppm (s, 3H).

Using compound A16 as raw material, the following compound A17 was obtained by a synthetic route similar to that of compound A15.

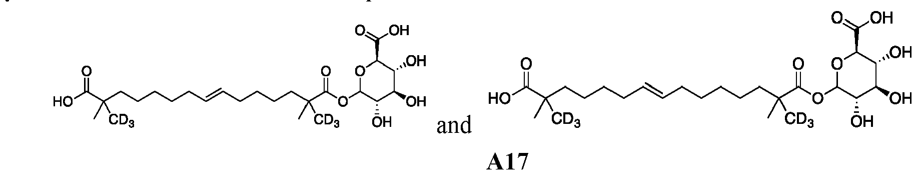

Compound A17: ¹H NMR (400 MHz, CD₃OD) δ 5.48 (d, J = 7.9 Hz, 1H), 5.37 - 5.41 (m, 2H), 3.82 (d, J = 9.4 Hz, 1H), 3.58 - 3.55 (t, 1H), 3.48 - 3.44 (t, 1H), 3.42-3.38(m, 1H), 2.01 - 1.94 (m, 4H), 1.60 - 1.44 (m, 4H), 1.39 - 1.21 (m, 10H), 1.19 (s, 3H), 1.15 (s, 3H) ppm. LR-ESI-MS m/z calcd for C₂₅H₃₆D₆O₁₀ [M-H]⁻: 507.2, found 507.2.

### Example 2: Evaluation of biological activity in vitro

### (1) Inhibitory activity of compounds against ACLY citrate cleavage enzyme

The ACLY-catalyzed citric acid cleavage process requires the consumption of one molecule of ATP to activate citric acid. Based on this reaction principle, the inventors utilized an ADP-Glo assay kit to detect the amount of ATP converted to ADP in response to the catalytic activity of ACLY. After the establishment of the activity measurement system and optimization of the reaction conditions, the inventors evaluated the ACLY inhibitory activity of the obtained novel ACLY small molecule inhibitors.

Activity test protocol: Human-derived ACLY protein was purchased from Beijing Yiqiao Shenzhou Technology Co., Ltd (Item No. 11769-H07B), and ADP-Glo assay kit was purchased from Promega (Item No. V9102). The final system of the reaction was shown in Table 1. The total volume of the reaction was 5 µL, which consisted of a mixture of three parts: 1 µL of 2% DMSO, 2 µL of the substrate mixture, and 2 µL of the enzyme (final concentration of 25 nM). Then 1 µL of different concentrations of compounds were added for incubation. Reaction conditions: 30 min, 37 °C. Then 2.5 µL of ADP Reagent from ADP-Glo Kit was used, mixed well and reacted for 2 h. 5 µL of ADP Detection was added, centrifuged and mixed well. Then the full wavelength value was read from Envision multifunctional plate reader.

**Table 1. Enzymatic reaction system**

| Component | Name | Concentration | Notes |
|---|---|---|---|
| Reaction buffer | Tris (pH=8.0) | 40 mM | |
| | MgCl₂ | 10 mM | |
| | DTT | 5 mM | |
| Substrate mixture | Citrate | 50 µM | dissolved in reaction buffer |
| | CoA | 15 µM | |
| | ATP | 5 µM | |

Results: Firstly, the cumulative amount of fluorescence intensity of the enzyme in 30 min reaction time (RFU/min) was calculated to represent the initial velocity of the enzyme, and then the ratio of the enzyme's velocity with the addition of compounds (vsample) to the enzyme's velocity without the addition of compounds (vDMSO) was calculated to be the percentage of activity for each concentration group of the samples (Activity rate %), with the formula: %Activity=(V(sample)/(v(DMSO))×100%. The logarithmic value of the concentration was plotted against the activity percentage, and then a non-linear regression was used to calculate the fitted curve. The software GraphPad Prism 8.0 (formula log(inhibitor) vs. response -- Variable slope) was used to fit the curve and calculate the IC₅₀ value, and the results are shown in the table below.

**Table 2. IC50 values for inhibition of ACLY enzyme activity by some compounds (mean±SD)**

| Compound Name | IC₅₀ (µM) |
|---|---|
| A1 | 42.3±6.5 |
| A2 | 104.5±7.1 |
| A3 | 85.4±9.8 |
| A4 | 53.9±13.8 |
| A5 | 16.7±1.8 |
| A6 | 25.5±6.8 |
| A7 | 12.0±2.2 |
| A8 | 21.3±9.7 |
| A9 | 25.8±9.2 |
| A10 | 9.6±5.1 |
| A11 | 40.19±2.9 |
| A12 | 56.90±4.7 |
| A13 | 5.31±1.2 |
| A14 | 5.63±1.2 |
| A15 | 36.7±0.8 |
| ETC-1002-CoA | 10.56±2.9 |

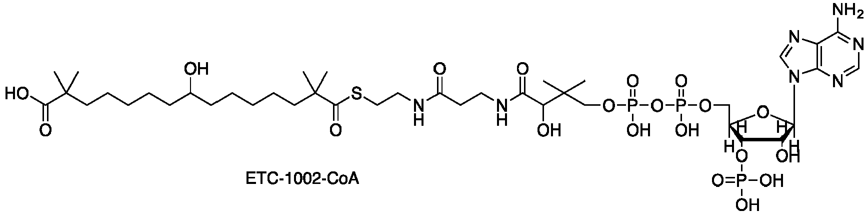

Above is the structural formula of the positive control ETC-1002-CoA.

### (2) Evaluation of activity of the compounds for inhibition of de novo synthesis of lipid in primary hepatocytes

ACLY is responsible for the cleavage of mitochondria-derived citric acid to generate acetyl coenzyme A, which serves as a key raw material for cellular lipid synthesis, and inhibition of ACLY activity significantly reduces the de novo synthesis of lipids (triglycerides and cholesterol). To further evaluate the functional changes induced by the inhibition of ACLY by the tested compounds, the inventors isolated primary mouse hepatocytes and evaluated the ability of the de novo synthesis of lipids using [1,2-¹⁴C]-sodium acetate as a substrate tracer, while compound ETC-1002 was used as a positive control.

### Test protocol:

Primary hepatic cell isolation and walling: the mouse liver was lavaged by calcium-free perfusate, then digested using collagenase and mouse primary hepatocytes were obtained by density gradient centrifugation, and were inoculated in 6-well plates at a density of 3×10⁵/ml (0.2% gelatin was coated before inoculating). After inoculating, the cells were cultured and adhered for 6 h. The medium was changed to serum-free LG-DMEM (containing 2x double antibody) and starved the cells overnight.

Isotope tracing: the next day, the medium was switched to serum-free LG-DMEM containing 10 nM insulin and the corresponding concentration of compounds (1950 µL, 4 replicate wells per group); 0.1 µCi of [1,2-¹⁴C]-acetate serum-free medium was added to each well, and the plates were then incubated for 4 h in the incubator.

Lipid saponification: Add 1.6 mL of ice PBS to each well for washing 3 times, shake the plate to dry and add 600 µL of 0.5 M KOH to each well to lysis for 1 h. Pipette 480 µL into a glass test tube with 400 µL of 20% KOH (methanol was used as solvent), shake and mix well, and then place it in a water bath at 95 °C to saponify for 3 h. The remaining lysate was used for the determination of the concentration of proteins. Add 400 µL of petroleum ether to each tube, pipette 5-6 times and centrifuge at 2500 rpm for 5 min, take 350 µL of the upper layer of the clear solution to the EP tube, and then add 400 µL of petroleum ether to repeat the extraction for 2 times (a total of 3 times). After complete transfer of the supernatant, 200 µL of water and 400 µL of 5N sulfuric acid were added to each tube for acidification, and the mixture was shaken and mixed. Add 400 µL of petroleum ether to each tube, pipette 5-6 times and centrifuge at 2500 rpm for 5 min, take 350 µL of the upper layer of the clear solution to the EP tube, then add 400 µL of petroleum ether and repeat the extraction for 2 times (a total of 3 times). Place in a fume hood and evaporate the petroleum ether overnight.

Isotope participation quantification: 1700 µL of scintillation solution was added to each tube and read.

Data processing and analysis: Firstly, the isotopic reference (R control, unit: CCPM/mg protein) of free fatty acids and cholesterol was calculated for the Control group of untreated mouse primary hepatocytes within 4 hours, which represented the basal synthesis. The percent inhibition of lipid synthesis was then calculated for each concentration group of the sample.

The inhibition rates on de novo synthesis of triglyceride and cholesterol after treatment of primary hepatocytes with some compounds (12.5 µM) are shown in the table below.

**Table 3. Inhibition rate (mean±SD) of lipid synthesis in primary hepatocytes by some compounds**

| Compound Name | Lipid synthesis inhibition (%) |
|---|---|
| Control | 0±19.1 |
| A2 | 80.9±5.9 |
| A3 | 71.2±4.3 |
| A4 | 78.2±4.3 |
| A5 | 56.6±7.6 |
| A6 | 68.7±20.9 |
| A8 | 21.6±1.3 |
| A9 | 30.9±4.2 |
| A10 | 21.1±11.7 |
| A12 | 38.7±11.4 |
| A15* | 58.2±7.4 |
| ETC-1002 | 46.3±15.3 |

| | |
|---|---|
| * represents that the compound was used at a concentration of 8 µM. | |

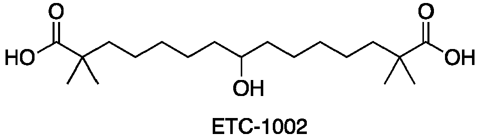

Above is the structural formula of the positive control ETC-1002.

### (3) Dependence of the lipid synthesis inhibitory activity of compound A3 on ACLY

Test protocol: In order to further investigate the dependence of the inhibitory effect of the series of compounds on de novo lipid synthesis on ACLY, the inventors chose ACLY knockdown primary hepatocytes for further evaluation of de novo lipid synthesis, while compound ETC-1002 was used as a positive control. Specifically, the experimental protocol was as above, and 0.1 µCi/well [¹⁴C]-citric acid was used for tracing.

Experimental results: In primary hepatocytes from WT mice, both ACLY small molecule inhibitor ETC-1002 and compound A3 significantly inhibited the de novo synthesis of triglycerides (A in Figure 1) and cholesterol (B in Figure 1) in cells using [¹⁴C]-citric acid as substrate. However, in ACLY knockdown hepatocytes, the inhibitory effects of the above two compounds on the de novo synthesis of triglycerides and cholesterol were significantly attenuated or even the inhibitory effect was not manifested (Figure 1).

### Example 3: Efficacy of compounds A3 and A4 in ameliorating hypercholesterolemia

### (1) Amelioration of hyperlipidemia and hypercholesterolemia in golden hamster by compounds A3 and A4

Dosing regimen: The hyperlipidemia model of golden hamster is recognized as one of the ideal animal models for studying hyperlipidemia drug therapy (pharmacodynamic evaluation). In order to preliminarily investigate the existence of potential regulatory effects of the candidate compounds on hepatic lipid metabolism homeostasis, the high-fat and high-cholesterol diet-induced hyperlipidemia model of golden hamster was utilized for preliminary pharmacodynamic evaluation. The specific protocol was as follows. 56 golden hamsters, males, were modeled on high-fat and high-cholesterol diet (Research diet, item number C11953) for two weeks, and 8 golden hamsters were reserved as blank control group. Subsequently, group administration (7 groups, n=8) was performed, i.e., model control group, positive control ETC-1002 30 mg/kg group, compound A3 10 mg/kg and 30 mg/kg group, compound A4 10 mg/kg and 30 mg/kg group, and blank control group. The compounds in each group were configured with saline (0.9% NaCl) and administered at a frequency of 1 time/day for a 7-day dosing cycle.

Indicator test: The body weight and food intake of golden hamsters were closely monitored during the administration period. After 7 days of administration, blood was collected from the orbital region of each group (starved overnight for about 16 hours), centrifuged to obtain serum, and stored in cryopreservation, and then total cholesterol and LDL cholesterol were detected in the serum. The relative amount of the relevant indexes was calculated as follows: Relative amount (100%) = the amount in the administered group/the amount in the model control group* 100%.

Results: Compared with the control group, after 1 week of compound administration: 1) both dose groups of compound A3 could significantly reduce the serum total cholesterol and LDL cholesterol of the golden hamster, and the reduction of the high-dose group was comparable to that of the positive control ETC-1002 30 mg/kg dose group; 2) the serum total cholesterol and LDL cholesterol of the compound A4 10 mg/kg administration group did not change significantly, and the 30 mg/kg administration group could significantly reduce the serum total cholesterol and LDL cholesterol (Figure 2). In addition, it was found that during the administration of Compound A4, there was a significant decrease in body weight, a significant decrease in food intake, and intolerance phenomena such as slowed activity.

Taken together, these results suggest that compound A3 is more effective and safe than compound A4 in improving hypercholesterolemia, and further suggest that the trans configuration of the double bond is important for the therapeutic effect of hypercholesterolemia.

### (2) Amelioration of compound A3 on atherosclerosis in ApoE^{-/-} mice

Dosing regimen: Hypercholesterolemia is an important trigger for the development of atherosclerosis. On the premise of seeing that Compound A3 has significant improvement on hyperlipidemia and hypercholesterolemia, the inventors further found that Compound A3 can significantly slow down the occurrence of atherosclerosis in ApoE^{-/-} mice. The specific scheme is as follows. 60 ApoE^{-/-} mice, males, were equally divided into 6 groups (n=10), of which 5 groups were fed with high cholesterol chow (Research diet, item no. D12079B) for modeling, and 1 group was set aside as a blank control group, while long-term administration of the drug was carried out, i.e., the model control group, the positive control ETC-1002 30 mg/kg group, and the compounds A3 15 mg/kg and 30 mg/kg and 60 mg/kg groups, and blank control group. The compounds in each group were configured with saline (0.9% NaCl) and administered at a frequency of 1 time/day for a 24-week dosing cycle.

Indicator test: The body weight and food intake of ApoE^{-/-} mice were closely monitored during the administration period. After 24 weeks of compound administration in each group, the aortic vessels of mice were stripped and Sudan IV staining of aortic plaques was performed, and the plaque area was counted with Image-Pro Plus software.

RESULTS: The results in Figure 3 showed that, compared with the control group, all three dosage groups of compound A3 significantly reduced the area of aortic plaque in ApoE^{-/-} mice after 24 weeks of compound administration (the right graph is the statistical graph of the left graph), indicating that compound A3 significantly ameliorates the development of atherosclerosis induced by hypercholesterolemia

### Example 4: Evaluation of the efficacy of compound A3 in improving diet-induced non-alcoholic steatohepatitis

The pathogenesis of non-alcoholic steatohepatitis (NASH) has been studied in many studies, and in general, "lipid deposition is the foundation, inflammation is the core, and fibrosis is the key factor for the deterioration of the disease or the difficulty in controlling the disease". On the one hand, inhibition of ACLY activity in hepatocytes can effectively reduce de novo synthesis of hepatic lipid, and reduce the important factors driving the development of NASH; on the other hand, ACLY mediates epigenetic modifications of histone proteins (acetylation, succinylation, etc.), which triggers metabolic reprogramming of immune cells, and response to inflammatory stimuli and generation of ROS. Moreover, the inhibition of de novo synthesis of hepatic stellate cell can effectively reduce the activation of hepatic stellate cells (HSC). Based on the potential intervention in the three major features of NASH development (lipid accumulation, inflammation, and fibrosis), the inventors further evaluated the feasibility of Compound A3 for the amelioration of NASH.

### (1) Amelioration of compound A3 on NASH induced by high-fat, high-sugar, high-cholesterol diet in ob/ob mice

Dosing regimen: 16 ob/ob mice, males, 7-8 weeks, were equally divided into 2 groups (n=8) and fed with high-fat, high-sugar, high-cholesterol diet (Research diet, item number D09100310) for 8 weeks for modeling, and a group of wild-type mice from the same litter was set up as a blank control group. Long-term drug administration was started after 8 weeks of modeling and the groups were model control group, compound A3 30 mg/kg group, and blank control group. The compounds in each group were prepared in saline (0.9% NaCl) and administered at a frequency of 1 time/day for 10 weeks.

Indicator test: The body weight and feeding changes of *ob*/*ob* mice and blank control mice were closely monitored during the administration period. After 10 weeks of administration, the livers of each group were taken and tested for NASH indicators (HE staining, Sirius red staining, immunohistochemistry for inflammatory signals, etc.).

Results: *ob*/*ob* mice showed whitening and granulomatous sensation due to excessive lipid accumulation, which was significantly reversed by compound A3 administration (A in Fig. 4); furthermore, the inventors subjected the livers to pathological analysis: compared with the model control group, H&E staining study revealed that liver steatosis and ballooning were significantly reduced in the group with compound A3 administration (steatohepatitis, A in Fig. 4); macrophage CD68 staining study found that hepatic inflammatory cell infiltration was significantly reduced in the compound A3 administration group (inflammation, A in Fig. 4, B in Fig. 4); Sirius red staining study found that the degree of hepatic collagen deposition was significantly reduced in the compound A3 administration group (fibrosis, A in Fig. 4, C in Fig. 4). The above results suggest that compound A3 has the pharmacological activity to significantly improve diet-induced NASH in *ob*/*ob* mice.

### (2) Amelioration of Compound A3 on high-fat, high-sugar, high-cholesterol diet-induced NASH in crab-eating monkeys

The inventors further evaluated the therapeutic effect of Compound A3 on non-alcoholic steatohepatitis in crab-eating monkeys by long-term administration of Compound A3 in a non-human primate model.

Dosing regimen: 18 non-alcoholic steatohepatitis-eating crab monkeys, males, were divided into 2 groups (n=8-10), one group was set up as a model control group, and one group was set up as a drug administration group. The drug (20 mg/kg) was administered at a frequency of 1 time/day for 14 weeks while feeding a high-fat, high-sugar, high-cholesterol diet for 14 weeks.

Indicator tests: The body weights and food intake of the crab-eating monkeys were closely monitored during the administration period. After 14 weeks of compound administration, liver puncture was performed for pathologic analysis of NASH indicators.

Results: The results of liver puncture showed that long-term administration of compound A3 could significantly reduce the accumulation of hepatic lipids and the occurrence of inflammation and fibrosis in crab-eating monkeys, and the activity score of non-alcoholic steatohepatopathy (NAS) was significantly reduced after the administration of compound A3 (Figure 5). The above results indicate that compound A3 possesses pharmacological activity to significantly ameliorate non-alcoholic steatohepatitis in crab-eating monkeys.

### Example 5: Examination of Oral Pharmacokinetic Parameters of Compound A3

Previous studies have found that Compound A3 is significantly better than ETC-1002 in improving atherosclerosis, and this study will compare the oral drug exposure and related pharmacokinetic parameters of the two compounds.

Dosing regimen: 6 normal ICR mice, male, were divided into 2 groups (n=3), one group was orally given the positive control ETC-1002, and one group was orally given the tested compound A3, both administered at a dose of 30 mg/kg; blood was taken from the eye orbits at different time points before and after the administration of the drug, and the plasma drug concentration was detected.

RESULTS: The plasma drug concentrations were measured at different times and pharmacokinetic parameters were calculated for two compounds (Table 4). Examination revealed that the plasma exposure of the tested compound A3 was significantly higher than that of the positive control drug ETC-1002 (Cₘₐₓ: 13.4 µg/mL vs 3.81 µg/mL; AUC₀₋ₜ: 63.7 h*µg/mL vs 14.4 h*µg/mL, the former for compound A3).

**Table 4: Oral pharmacokinetic parameters of compound A3 and control ETC-1002**

| Subjects/parameters | Tmax (h) | Cmax (µg/mL) | AUC₀₋ₜ (h*µg/mL) | AUC_{0-∞} (h*µg/mL) | MRT_{0-∞} (h) | t_{1/2} (h) |
|---|---|---|---|---|---|---|
| Compound A3 | 0.25 | 13.4 | 63.7 | 74.2 | 8.01 | 5.65 |
| ETC-1002 | 0.25 | 3.81 | 14.4 | 15.6 | 6.81 | 4.45 |

The above test results indicate that the compounds of the present invention have ACLY inhibitory activity. Further in vitro and in vivo pharmacological activity evaluations reveal that representative compound A3 inhibits the de novo synthesis of lipids in primary hepatocytes, improve hyperlipidemia and hypercholesterolemia in golden hamsters and ApoE^{-/-} mice, and significantly improve NASH symptoms induced by high-fat, high-sugar, high-cholesterol diet in *ob*/*ob* mice and crab-eating Macaque. The research results also suggest that the in vivo activity of compounds with a double bond in the trans configuration is better than that of compounds with a double bond in the cis configuration; moreover, the plasma exposure of Compound A3 is more than 4 times higher than that of Bempedoic acid at the same oral dose, suggesting that compound A3 has better therapeutic effect.

In conclusion, the series of compounds in the presentinvention have good ACLY inhibitory activity and have shown good therapeutic effects in various metabolic disease pharmacodynamic models, which can be further developed as therapeutic agents for metabolic diseases such as hypercholesterolemia, non-alcoholic steatohepatitis and so on.

## Claims

1. Use of a compound represented by general formula (I), or a stereoisomer, enantiomer, diastereomer, racemate or pharmaceutically acceptable salt thereof, for the preparation of a medicament for the treatment of a metabolic disease; or for the preparation of a medicament for the treatment of atherosclerosis, 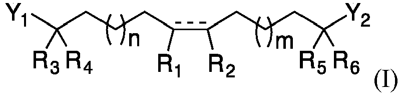
wherein - - - represents a double bond or a single bond;
m and n are each independently 1, 2, 3, 4, 5 or 6;
R₁, R₂, R₃, R₄, R₅ and R₆ are each independently H, C1-C4 alkyl, C2-C4 alkenyl, C2-C4 alkynyl, C3-C7 cycloalkyl, C3-C7 cycloalkenyl, phenyl or benzyl;
or R₁ and R₂, together with their adjacent carbon atoms, form a C3-C7 cycloalkyl or C3-C7 cycloalkenyl;
or R₅ and R₆, together with their adjacent carbon atom, form a C3-C7 cycloalkyl or C3-C7 cycloalkenyl;
or R₃ and R₄, together with their adjacent carbon atom, form a C3-C7 cycloalkyl or C3-C7 cycloalkenyl;
Y₁ and Y₂ are each independently selected from the group consisting of -OH, -COOH, - COCoA, and -COOR₇; wherein R₇ is methyl, ethyl or 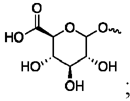 CoA is as follows:

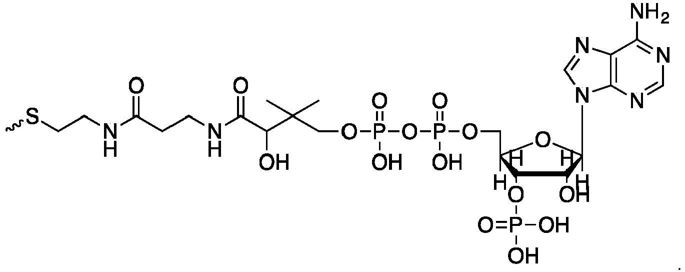

2. The use of claim 1, wherein the compound has a structure represented by general formula II:

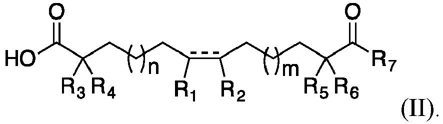

3. The use of claim 1, wherein the compound has a structure represented by general formula III:

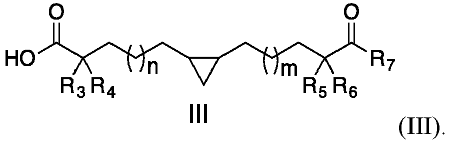

4. The use of claim 1, wherein the compound has a structure represented by general formula IV: 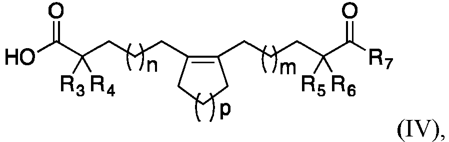 wherein p is 0, 1, 2 or 3.

5. The use of any one of claims 1 to 4, wherein the compound is a deuterated compound.

6. The use of any one of claims 1 to 5, wherein the compound is selected from the following group: 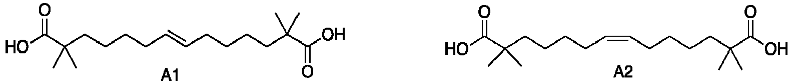 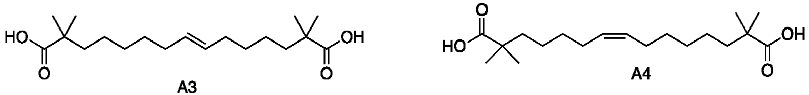 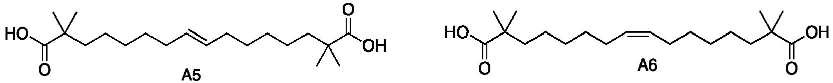 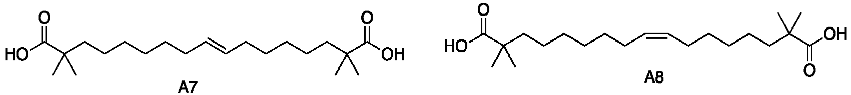 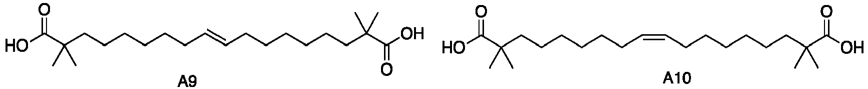 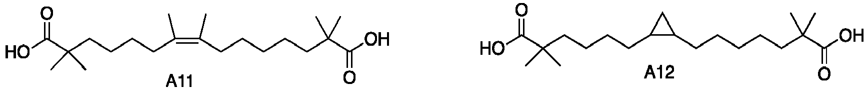 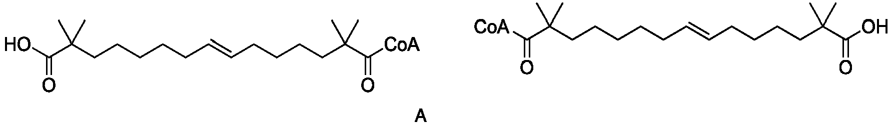 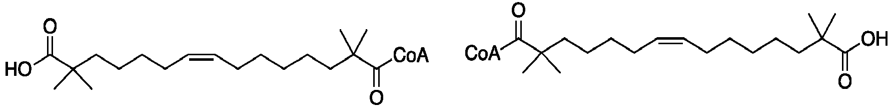 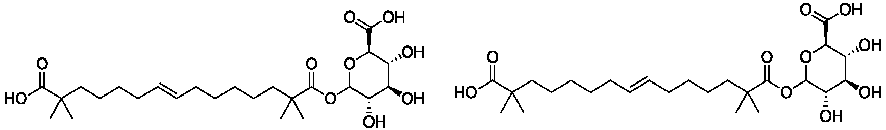 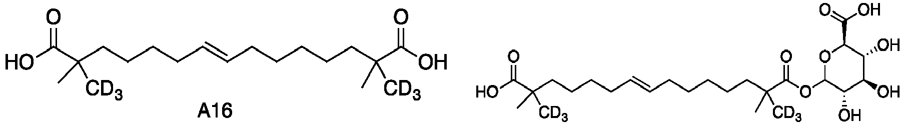 and

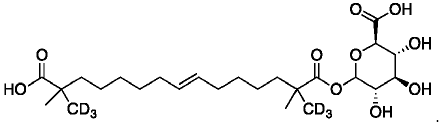

7. The use of claim 1, wherein the metabolic disease is selected from the group consisting of hypercholesterolemia, non-alcoholic steatohepatitis (NASH), and liver fibrosis.

8. A compound represented by general formula (I), or a stereoisomer, enantiomer, diastereomer, racemate or pharmaceutically acceptable salt thereof, 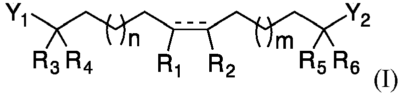
wherein - - - represents a double bond or a single bond;
m and n are each independently 1, 2, 3, 4, 5 or 6;
R₁, R₂, R₃, R₄, R₅ and R₆ are each independently H, C1-C4 alkyl, C2-C4 alkenyl, C2-C4 alkynyl, C3-C7 cycloalkyl, C3-C7 cycloalkenyl, phenyl or benzyl;
or R₁ and R₂, together with their adjacent carbon atoms, form a C3-C7 cycloalkyl or C3-C7 cycloalkenyl;
or R₅ and R₆, together with their adjacent carbon atom, form a C3-C7 cycloalkyl or C3-C7 cycloalkenyl;
or R₃ and R₄, together with their adjacent carbon atom, form a C3-C7 cycloalkyl or C3-C7 cycloalkenyl;
Y₁ and Y₂ are each independently selected from the group consisting of -OH, -COOH, - COCoA, and -COOR₇; wherein R₇ is methyl, ethyl or 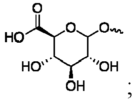 CoA is as follows:

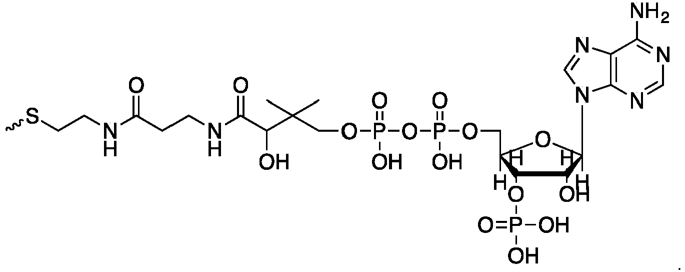

9. The compound of claim 8, or the stereoisomer, enantiomer, diastereomer, racemate or pharmaceutically acceptable salt thereof, wherein the compound has a structure represented by general formula II, III or IV: 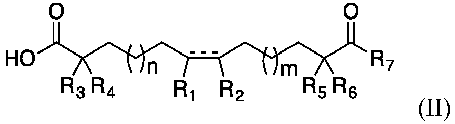 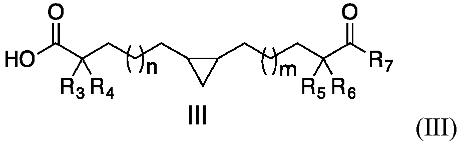 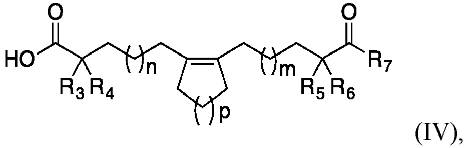 wherein p is 0, 1, 2 or 3.

10. The compound of claim 8 or 9, or the stereoisomer, enantiomer, diastereomer, racemate or pharmaceutically acceptable salt thereof, wherein the compound is a deuterated compound.

11. The compound of any one of claims 8 to 10, or the stereoisomer, enantiomer, diastereomer, racemate or pharmaceutically acceptable salt thereof, wherein the compound is selected from the following group: 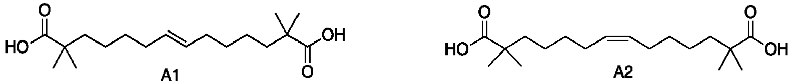 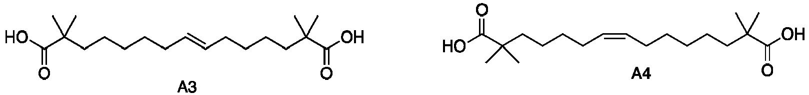 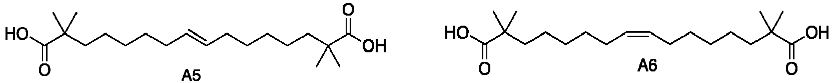 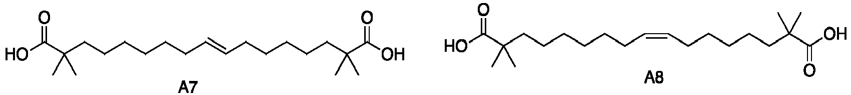 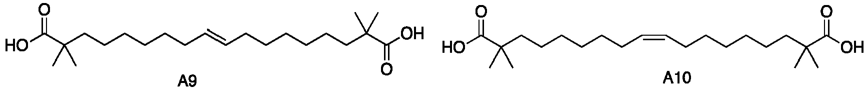 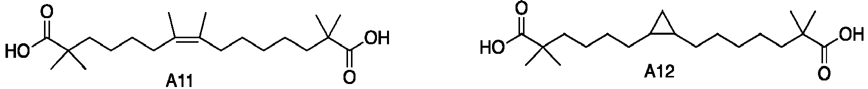 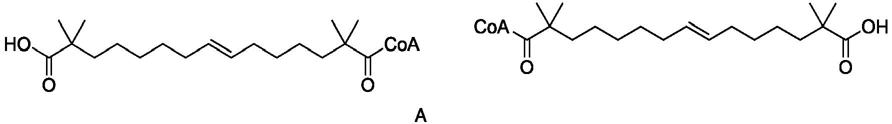 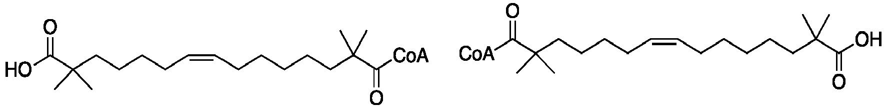 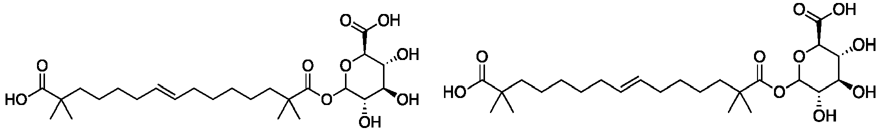 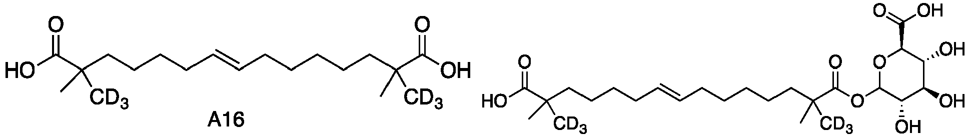 and

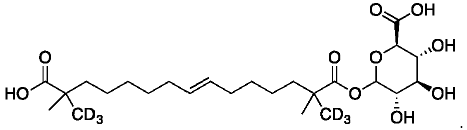

12. A pharmaceutical composition comprising the compound of any one of claims 8-11, or the stereoisomer, enantiomer, diastereomer, racemate or pharmaceutically acceptable compound thereof; and
a pharmaceutically acceptable salt.
